# EUROPEAN PATENT APPLICATION

(11) **EP 1 609 437 A1**
(43) Date of publication of application: **28.12.2005**
(21) Application number: 04019488.8
(22) Date of filing: 17.08.2004
(51) Int. Cl.: A61C 13/00

(54) **Driving apparatus for three-dimensional scanning system and three-dimensional scanning system for computer-aided tooth modelling using the same**

(30) Priority: 24.06.2004 KR 2004047372
(71) Applicant: KCI Co., Ltd, Gangnam-gu Seoul (KR)
(72) Inventor: Park, Kang, Bungdang-ku Seongnam-si Kyungki-do (KR); Kang, Seok Jin, Jukjeon-dong, Yongin-si Kyungki-do (KR); Kwon, Ha Ja, Jukjeon-dong, Yongin-si Kyungki-do (KR)
(74) Representative: Hano, Christian

(57) **Abstract**

The 3D scanning system includes an image detector which extracts image data of a tooth plaster model, a four-axis drive which alters the position and posture of the tooth plaster model measured by the image detector, and a controller which controls the drive to alter the position and posture of the tooth plaster model, in order to measure the tooth plaster model using the image detector. The four-axis drive secures three kinds of the degrees of freedom such as tilt, rotation and main rotation with respect to a table jig on which a tooth plaster model is mounted and one kind of the degree of freedom such as linear driving for position setting. Accordingly, the posture of the tooth plaster model can be transformed into any posture, to thereby heighten a scanning automation rate, and to thus achieve measurement efficiency.

## Description

### BACKGROUND OF THE INVENTION

### 1. Field of the Invention

The present invention relates to a driving apparatus for a three-dimensional scanning system, and a non-contact scanning system for computer-aided tooth modelling using the same, and more particularly, to a three-dimensional scanning system driving apparatus, and a non-contact computer-aided tooth modelling scanning system using the same, which secures three kinds of the degrees of freedom such as tilt, rotation and main rotation with respect to a table jig on which a tooth plaster model is mounted and one kind of the degree of freedom such as linear driving for position setting, to accordingly transform the posture of the tooth plaster model into any posture, and to thereby heighten a scanning work efficiency.

### 2. Description of the Related Art

In general, an orthodontic treatment for correcting a tooth or teeth that is carried out in a dental clinic means a series of procedures of dealing with prevention and correction of a functional disorder generally entailing the straightening of crooked teeth or the correcting of a poor bite, or malocclusion (physiologically unacceptable contact of opposing dentition) and of an esthetic disorder generally entailing the correcting of a protruding chin, a snaggle tooth, an inturned tooth, or the like, which are caused due to irregularities of the teeth.

In treating the functional and aesthetic disorders of the tooth or teeth which occur due to the irregularities of the teeth as described above, a three-dimensional scanning method is chiefly used for obtaining three-dimensional configuration data in order to obtain measure and analyze configuration of the tooth or teeth necessary for correction thereof.

The correcting treatment includes: the steps of manufacturing a patient's teeth plaster cast at a point in time of initializing the treatment and obtaining and measuring configuration data such as a location of the tooth, a gradient of the tooth, and the like; determining a proper method for correcting an irregular tooth alignment into a regular tooth alignment; and mounting a bracket for correction on the tooth to then correct the tooth.

However, the conventional method has a drawback in measuring the configuration data such as the location and gradient of the tooth, since the 3-dimensional (3D) configuration data is manually measured, thereby deteriorating accuracy and consuming lots of time and labor. In addition, the conventional method requires a large storage space and careful maintenance since a lot of plaster casts should be stored for years until the correcting treatment finishes.

To solve the aforesaid problems, there has been suggested a 3D scanning system which can scan a patient's tooth plaster model with a 3D scanner for a computer modeling, extract 3D configuration data on a computer, and store the plaster model in a computer data form.

In contrast to the conventional method in which the tooth plaster model is manually measured for dental correction, the 3D scanning system uses a 3D-coordinate measuring system in a manner that a plaster model is analyzed and data is processed in a computer, so that the configuration data necessary for the dental correction can be quickly and exactly measured within a shorter time than that of the conventional manual method.

Meanwhile, a tooth plaster model which becomes an object to be measured for the dental correction is very complex in shape and very rough on the surface thereof. In addition, the plaster model is almost uniform in its overall shape and size and uniform in its quality and color. In measuring the tooth plaster model having the aforementioned characteristics, there are parts which are important to the orthodontic treatment and other parts which are not important. It is desirable that a measuring time is as short as possible and the number of inputs of dentists is minimized until a measuring result is obtained.

Further, as for the measurement result, a measure accuracy should be high enough to be used for a dental treatment purpose, locations and intervals of measurement points should be selectable in order to generate a tooth model in computer graphics, and configuration data for medical use should be extracted from the measurement points.

An existing general-purpose 3D scanner is divided into a non-contact 3D scanner and a contact 3D scanner. The existing 3D scanner has the following features and problems in measuring the tooth plaster model having the above characteristics.

### 1. Non-contact 3D scanner

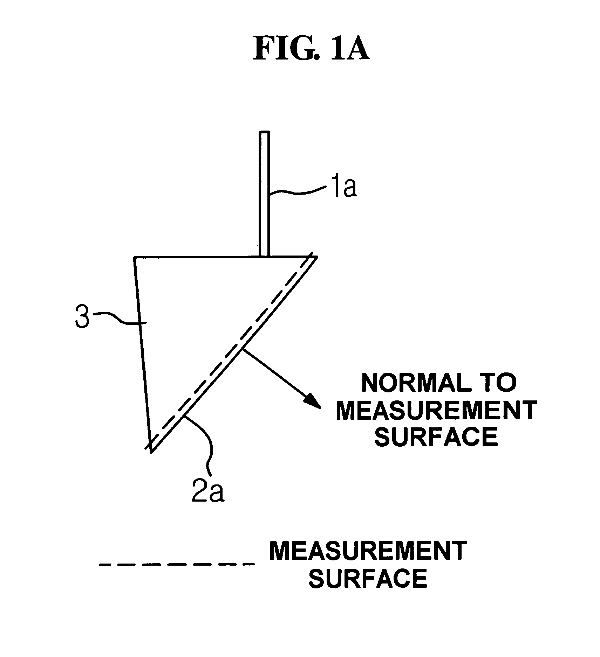

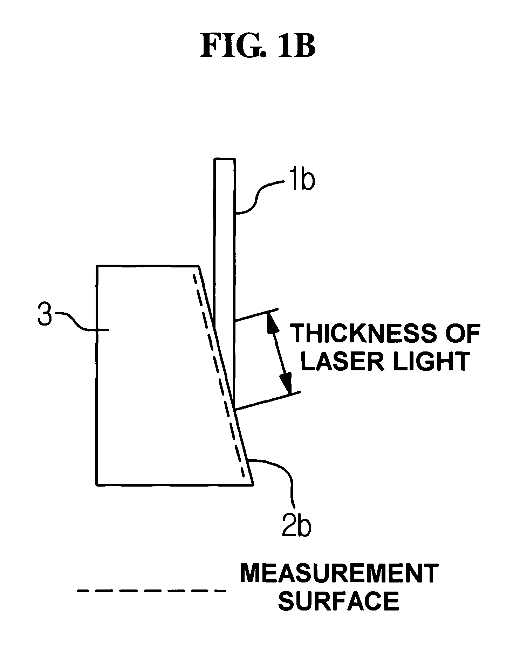

The non-contact 3D scanner is classified into a scanner which linearly drives a laser distance sensor in three-axis directions, and a scanner which uses a laser slit and a camera. The 3D scanner using the laser distance sensor has a difficulty in measuring a measurement surface 2a which is directed down to the tooth plaster model as shown in FIG. 1A and a vertical measurement surface 2b as shown in FIG. 1B. Therefore, a physical position of the object should be changed, the origin should be defined again and the object should be measured again. As a result, a measuring time is extended and a skillful technology is required. Further, a Z-axis should be moved along the shape of the object and an expensive reverse engineering program is required to extract the configuration data necessary for the orthodontic treatment.

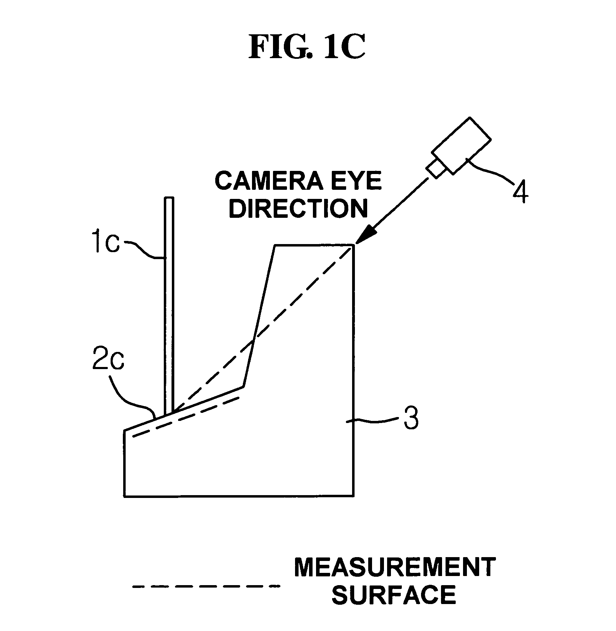

The scanner using the laser slit and camera is capable of performing a linear movement of one kind of the degree of freedom. However, this scanner disadvantageously generates a portion which is not able to be measured since when an object has a complex shape as shown in FIG. 1C, a range of vision of the camera 4 is hidden by the shape of the tooth plaster model 3 itself, and the scanner should use the expensive reverse engineering program to extract the configuration data necessary for the orthodontic treatment.

### 2. Contact 3D scanner

The contact 3D scanner is a contact scanner having three kinds of the degree of freedom, and obtains a 3D coordinate of a contact point by linearly moving a measuring probe in three directions of the x-axis, y-axis and z-axis and contacting the same to an object. This scanner cannot measure a curvature more minute than the probe in size and has a difficulty in measuring a downward-facing surface 1a of FIG. 1A. When measuring the downward-facing surface, the scanner changes the position of the object, adjusts the origin again, and then measures the object again. Accordingly, an operator should move the probe in person to measure the downward-facing surface, thereby delaying a measuring time and requiring an experienced skill. Additionally, the equipment is highly expensive and the expensive reverse engineering program should be used to extract the configuration data for the medical purpose.

As stated above, both the conventional non-contact and contact 3D scanners for measuring the tooth plaster model have drawbacks in that the complex-shaped plaster model is difficult to be measured, the skillful measuring technology is required and the expensive reverse engineering program is required to extract the configuration data.

The Applicant has solved a number of problems caused when the tooth plaster model is scanned, and proposed a 3D scanner system for computer-aided tooth modelling which can efficiently scan a tooth plaster model and produce a computer-aided model without having a separate reverse engineering program, in Korean Patent No. 382,905.

The non-contact 3D scanner of Korean Patent No. 382,905 has two kinds of the degree of freedom such as rotation and tilting with respect to a turntable supporting a tooth plaster model, and one kind of the degree of freedom such as linear movement for setting the position of a model by a drive composed of three axes. However, since the 3D scanner determines the position of a tooth plaster model by two kinds of the degree of freedom, it has a difficulty in measuring part of the surface contacting the tongue in a molar tooth which is the most difficult to measure. Accordingly, a number of scan paths are added unnecessarily to thereby cause an increase of a scanning time, which will be described in detail at a later time.

Also, a method of laser scanning a tooth plaster model and producing a digital image of the tooth plaster model is disclosed in Am. J. Orthodontics 110: 365-369 of Kuroda, in 1996. This method is also disclosed in U.S. Patent No. 5,605,459.

Methods of manipulating a digital image in order to design dental teeth aligning equipment are disclosed in U.S. Patent Nos. 5,533,895, 5,474,448, 5,454,717, 5,447,432, 5,431,562, 5,395,238, 5,368,478, and 5,139,419 which have been assigned to Ormco Corporation.

A method of making a tooth into a digital image and determining an optimal bracket alignment for dental equipment is disclosed in U.S. Patent No. 5,011,405. A method of laser scanning a tooth plaster model and making a 3D model is disclosed in U.S. Patent No. 5,338,198. A method of laser scanning a tooth model and milling a tooth frame is disclosed in U.S. Patent No. 5,452,219. Also, a digital computer-aided method of manipulating a tooth contour is disclosed in U.S. Patent Nos. 5,607,305 and 5,587,912.

However, the above-described prior art has not solved the above problems effectively in measuring a surface contacting the tongue in a molar tooth.

Also, the existing scanning methods for the existing 3D scanners are classified into cases where an object to be measured is fixed and scanned by a measuring unit which is mounted on a three-axis robot, where an object to be measured is made to rotate on a one-axis and scanned by a measuring unit which performs a one-axis linear movement, and where an object to be measured is mounted on a multi-axis robot and scanned by a number of measuring units. However, the above-described defects are recognized as scanning problems.

### SUMMARY OF THE INVENTION

To solve the above problems, it is an object of the present invention to provide a driving apparatus for a three-dimensional (3D) scanning system and a non-contact four-axis 3D scanning system for a computer-aided tooth plaster model using the same, in which the posture of a tooth plaster model is able to be transformed into any posture by a four-axis drive which secures three kinds of the degree of freedom such as tilt, rotation and main rotation and one kind of the degree of freedom such as linear movement, with respect to a table jig on which a tooth plaster model is mounted.

It is another object of the present invention to provide a 3D scanning system for a computer-aided tooth model, which enables a camera to transform any portion of a tooth plaster model including a surface contacting the tongue in a molar tooth into a posture which can be measured by rotation of a main rotation drive with respect to a tilting drive, to thereby enabling a scanning automation.

It is still another object of the present invention to provide a 3D scanning system for a computer-aided tooth model, which minimizes postures requiring manual measurement when a tooth plaster model is measured, increases postures enabling automatic measurement at maximum, reduces a scan path and shortens a scan time, to thereby heighten a scanning work efficiency.

It is yet another object of the present invention to provide an automated 3D scanning system which automatically changes the posture of an object to be measured by a drive, based on the origin initially set at the time of scan work without having an assistance of a skillful operator, to thereby shorten a measurement time remarkably.

It is still yet another object of the present invention to provide an automated 3D scanning system which automatically transforms the posture of a tooth into a desired position and posture, to thereby obtain measurement data whose accuracy can be guaranteed by only one-time scanning.

To accomplish the above object of the present invention, according to an aspect of the present invention, there is provided a driving apparatus for a three-dimensional (3D) scanning system, comprising: a first rotation drive which rotatably drives a table jig on which an object to be measured is mounted; a tilting drive which tilts the first rotation drive; a second rotation drive which rotates the tilting drive independently of the first rotation drive; and a linear drive which linearly moves the second rotation drive.

According to another aspect of the present invention, there is also provided a driving apparatus for a three-dimensional (3D) scanning system, comprising: a tilting bed which rotatably supports a table jig on which an object to be measured is mounted, around a first rotation center axis and includes first and second tilting supports which are vertically installed on both ends thereof; a rotation motor which is installed in the tilting bed for rotatably driving the table jig; a main rotation bed which includes first and second main rotation supports which are vertically installed in correspondence to the first and second tilting supports on both ends thereof and supports first and second tilting center axes extended from the first and second tilting supports so as to be tilted; a tilting motor which is installed in the main rotation bed and tilts the tilting bed; a support frame which rotatably supports the main rotation bed around a second rotation center axis; a linear drive bed on which the support frame is installed; a main rotation motor which is installed on the linear drive bed and rotatably drives the main rotation bed; and a linear drive which linearly moves the linear drive bed, wherein the table jig, the tilting bed and the main rotation bed are independently driven, respectively.

According to still another aspect of the present invention, there is also provided a three-dimensional scanning system for a computer-aided tooth modelling, comprising: a table jig on which a tooth plaster model is mounted; a laser light source which irradiates laser light to the tooth plaster model; a camera which photographs light reflected by the laser irradiated from the laser light source; a drive having a four axes to alter position and posture of the tooth plaster model; and a controller which controls the four-axis drive to alter the position and posture of the tooth plaster model, and signal-processes the image signal photographed by the camera to produce configuration data, wherein the four-axis drive comprises: a first rotation drive which rotatably drives a table jig on which an object to be measured is mounted; a tilting drive which tilts the first rotation drive; a second rotation drive which rotates the tilting drive independently of the first rotation drive; and a linear drive which linearly moves the second rotation drive.

According to yet another aspect of the present invention, there is also provided a three-dimensional (3D) scanning system for a computer-aided tooth modelling, comprising: a table jig on which a tooth plaster model is mounted; a tilting bed which rotatably supports the table jig around a first rotation center axis, and includes first and second tilting supports which are vertically installed on both ends thereof; a rotation motor which is installed in the tilting bed for rotatably driving the table jig; a main rotation bed which includes first and second main rotation supports which are vertically installed in correspondence to the first and second tilting supports on both ends thereof and supports first and second tilting center axes extended from the first and second tilting supports so as to be tilted; a tilting motor which is installed in the main rotation bed and tilts the tilting bed; a support frame which rotatably supports the main rotation bed around a second rotation center axis; a linear drive bed on which the support frame is installed; a main rotation motor which is installed on the linear drive bed and rotatably drives the main rotation bed; a linear drive which linearly moves the linear drive bed; a laser light source which irradiates laser light vertically when the tooth plaster model is located at the origin; a camera which photographs laser light reflected from the tooth plaster model; and a controller which controls the motors to alter the position and posture of the tooth plaster model, and signal-processes the image signal photographed by the camera to produce configuration data, wherein the table jig, the tilting bed, the main rotation bed and the linear drive bed are independently driven, respectively.

In this case, the first and second tilting center axes are identical to each other and simultaneously are horizontal with respect to the linear drive axis in the linear drive. The linear drive axis is perpendicular to the first rotation center axis in the table jig. The second rotation center axis in the main rotation bed is positioned on the same line as the first rotation center axis when the tilting bed and the main rotation bed are parallel with each other.

In the system according to the present invention, when the camera is positioned on the upper portion of the laser light source in the same direction as the linear drive axis of the linear drive bed with a predetermined distance from the laser light source, a surface contacting the tongue in a molar tooth of the tooth plaster model is measured in the state where the tilting center axis of the tilting bed is position-set in the direction which is same as a light reception direction of the camera or close to the light reception direction of the camera and then the tilting bed is tilted.

Thus, the present invention can measure a surface contacting the tongue in a molar tooth which is the most difficult to measure in a tooth plaster model, with an automatic measurement algorithm, to thereby accomplish an automatic measurement for almost all portions of the tooth plaster model.

As described above, the present invention actively transforms and measures position and posture of a tooth plaster model using a four-axis drive when the tooth plaster model for dental correction is three-dimensionally scanned, and thus provides an effect of measuring most positions of the tooth plaster model to be measured accurately without having an assistance of a skilled operator and quickly through a minimized scanning path.

Also, the present invention enables posture transformation freely and establishes a posture to be measured according to the shape and size of a tooth, that is, a scanning path. Accordingly, the present invention can measure a tooth plaster model at an optimal posture for the tooth and reduce unnecessary scanning paths greatly, to thereby reduce an amount of doubled scan data and enable data acquisition of a molar tooth more accurately.

### BRIEF DESCRIPTION OF THE DRAWINGS

The above and other objects and advantages of the present invention will become more apparent by describing the preferred embodiments thereof in detail with reference to the accompanying drawings in which:
FIGs. 1A to 1C are views for explaining a general measurement impossibility angle according to an irradiation angle of a laser measurement beam and the posture of an object to be measured, respectively;
FIGs. 2A and 2B are photos showing the upper and lower jaws of a tooth plaster model viewed from the rear side, respectively;
FIGs. 3A and 3B are photos showing a measurement possibility posture with respect to the lateral side of the tongue in the mouth for a tooth plaster model, respectively;
FIG. 4 is a schematic view showing the whole configuration of a four-axis three-dimensional scanning system for computer-aided modelling a tooth or teeth according to the present invention;
FIG. 5 is an enlarged perspective view of a four-axis drive in the present invention system shown in FIG. 4;
FIG. 6 is a view for explaining positional movement and posture alteration of a table jig in the four-axis drive of FIG. 5; and
FIGs. 7A and 7B are views for comparatively explaining positional movement and posture alteration of a table jig of a conventional three-axis drive and the four-axis drive according to the present invention, respectively.

### DETAILED DESCRIPTION OF THE INVENTION

Preferred embodiments of the present invention will be described with reference to the accompanying drawings.

FIGs. 1A to 1C are views for explaining a general measurement impossibility angle according to an irradiation angle of a laser measurement beam and the posture of an object to be measured, respectively.

Referring to FIGs. 1A to 1C, problems which occur in measuring a tooth plaster model using a conventional three-dimensional (3D) scanner will be described below in detail.

A conventional 3D scanner cannot easily measure a tooth plaster model 3 in the case that laser slit light 1a does not reach a measurement surface 2a since the measurement surface 2a of the tooth plaster model 3 is directed downwards with respect to the scanning direction of the laser slit light 1a as shown in FIG. 1A, in the case that laser slit light 1b irradiated to a measurement surface 2b becomes too thick since the measurement surface 2b and the laser slit light 1b are too inclined as shown in FIG. 1B, and in the case that laser slit light 1c irradiated to a measurement surface 2c is hidden by other portions of an object to be measured to thus make the laser slit light 1c invisible by a camera 4.

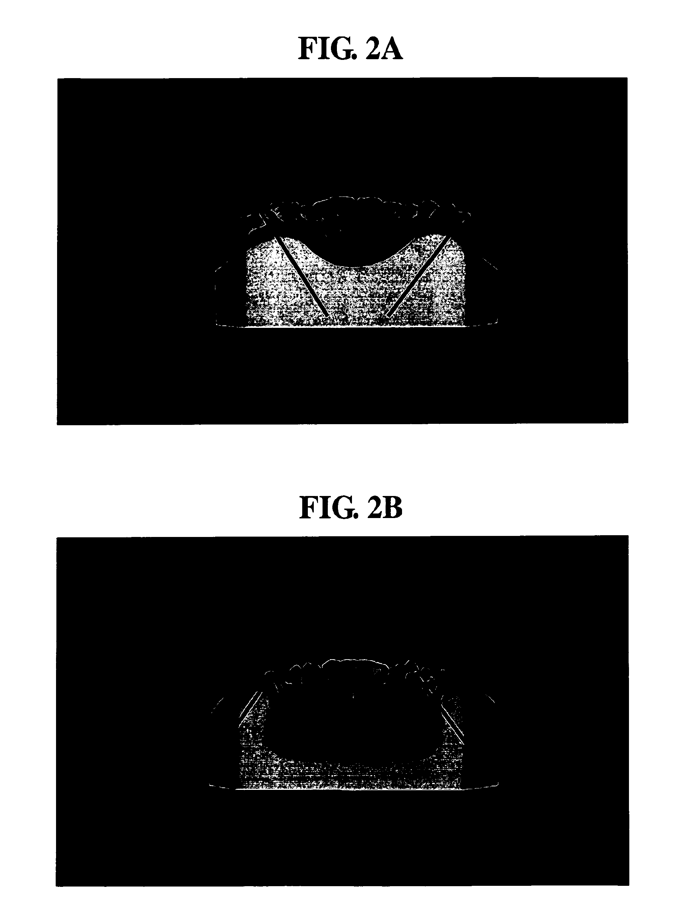

Teeth are classified into the upper jaw and the lower jaw, and also classified into a portion visible when a person laughs with the mouth opened, that is, a lip-side portion, and a rear-side where the tongue is positioned, that is, a tongue-side portion. In general, the upper jaw teeth are tilted toward the lip-side portion (outwards) as shown in FIG. 2A, and the lower jaw teeth are tilted toward the tongue-side portion (inwards) as shown in FIG. 2B, when viewed from the rear side on the basis of the tongue-side portion.

FIGs. 2A and 2B are photos showing the upper and lower jaws of a tooth plaster model viewed from the rear side, respectively.

In the case of the upper jaw where teeth are tilted outwards as shown in FIG. 2A, laser slit light can reach and a camera can recognize the reached laser slit light. However, in the case that teeth are tilted inwards as shown in FIG. 2B, laser slit light cannot reach, or the camera cannot recognize laser slit light although the laser slit light reaches.

In particular, the tongue-side surface in the molar teeth of the lower jaw shown in FIG. 2B is an example compositely corresponding to both cases incapable of measuring a tooth plaster model shown in FIGs. 1A and 1C. Thus, when such portions of a tooth plaster model are measured, an automatic measurement is not accomplished, but a manual measurement should be performed. Also, since the system does not support a measurement posture structurally, there remain portions which are not measured through even a manual measurement.

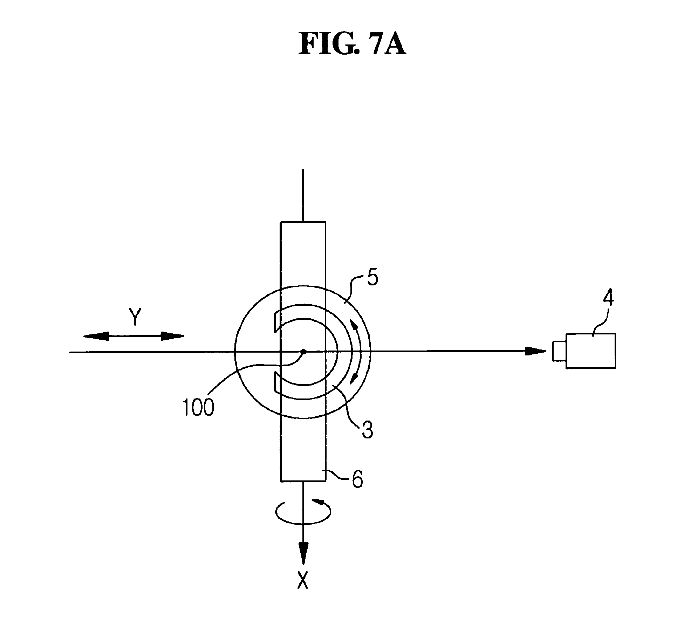

As shown in FIG. 7A, a conventional three-axis drive 3D scanner (refer to Korean Patent No. 382,905) includes a laser light source (not shown) which is disposed on the upper portion in the vertical direction from the origin 100, that is, in the Z-axis direction (not shown), and a data receiving camera 4 which is distant by a predetermined distance from the laser light source and tilted on the upper portion. Also, a tooth plaster model 3 which is an object to be measured moves in the same direction (Y-axis direction) as the light receiving direction of the camera 4 by a linear drive (not shown). A table jig 5 supporting the tooth plaster model 3 can rotate around the Z-axis. A tilting drive 6 which drives while tilting the table jig 5 around the X-axis is fixed in the direction (that is, the Y-axis) perpendicular to the direction where the laser light and the camera 4 are concatenated.

However, the three-axis driving scanner has not assumed measurable postures for a tongue-side portion (refer to FIG. 2B) in a molar tooth of the tooth plaster model formed by combining the cases of FIGs. 1A and 1C.

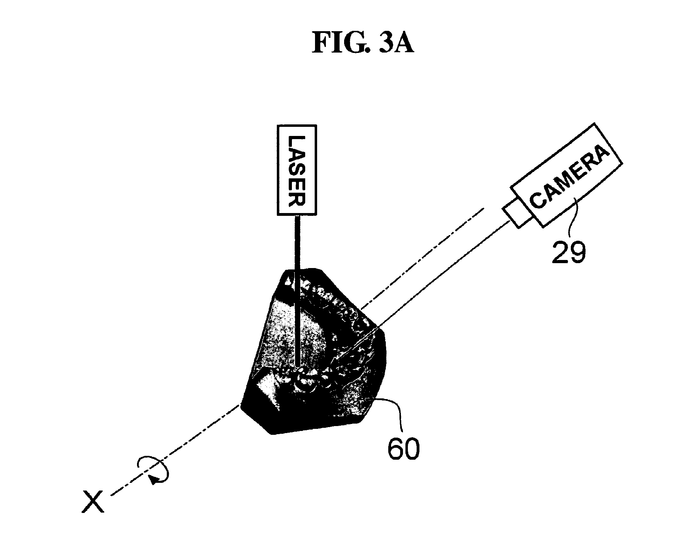

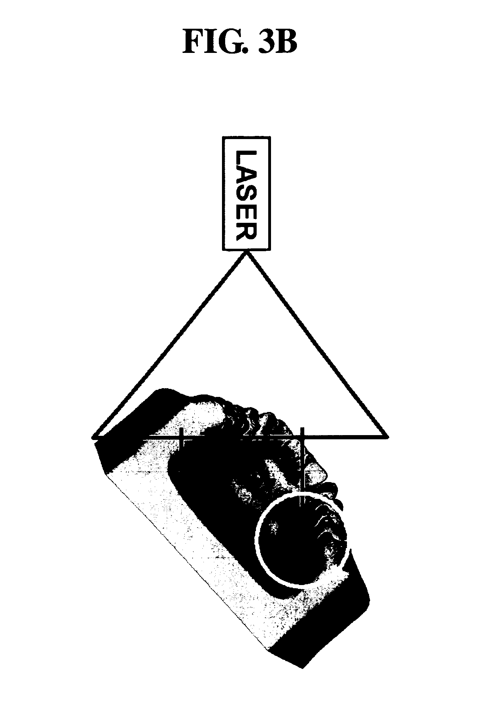

Thus, since laser slit light does not reach an inwardly slant portion as in the molar tooth tongue-side surface, due to the linearity of light, the postures as shown in FIGs. 3A and 3B are required so that laser light can reach the postures to be photographed by a camera.

Also, in order to solve the problem of FIG. 1A, a measurement surface 2a is turned to make the laser slit light 1 a scanned onto the measurement surface 2a at a nearly right angle, and in order to remove the problem of FIG. 1C, it is necessary to turn the tooth plaster model 3 so that the laser slit light 1 c scanned onto the measurement surface 2c can be viewed by the camera 4.

By the way, in order to realize the above-described postures, the rotational axis of the tilting drive 6, that is, the tilting axis should be in the same direction as the direction (which will be referred to as a camera direction) where the laser light and the camera 4 are concatenated, or set in the direction close to the camera direction. The conventional scanner cannot accomplish the required posture since the tilting drive 6 is fixed in the direction perpendicular to the direction concatenating the laser light and the camera 4.

Thus, the three-axis driving scanner has not performed a measurement of the molar tooth tongue-side surface of the tooth plaster model 3 by an automatic measurement algorithm, and should perform a partial measurement of a number of postures which can be measured by manual manipulation.

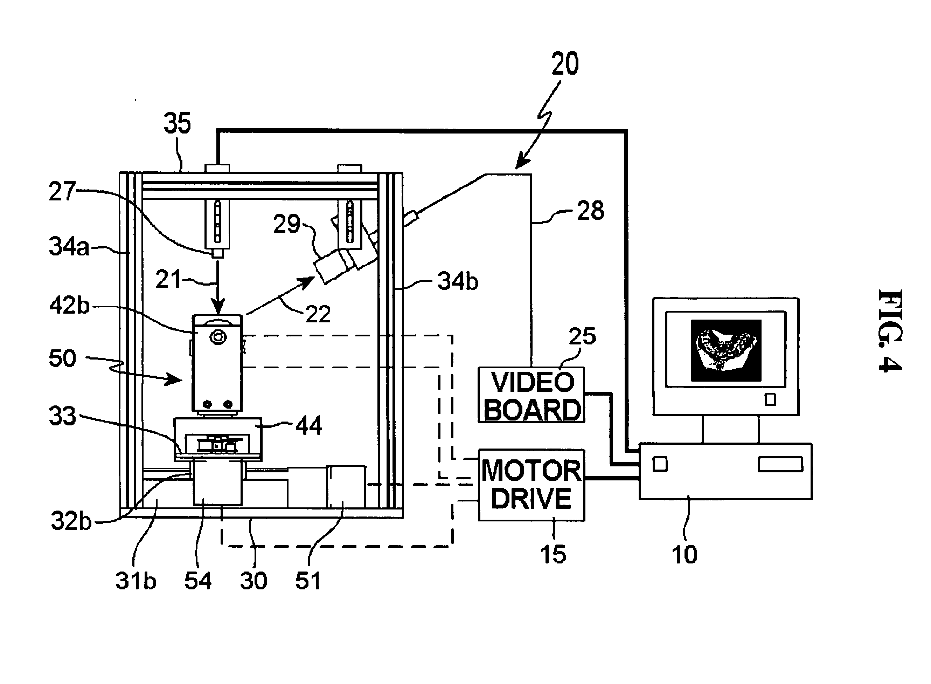

Hereinbelow, a 3D scanning system having a four kinds of the degree of freedom and four-axis drive according to the present invention will be described with reference to FIGs. 4 to 7B.

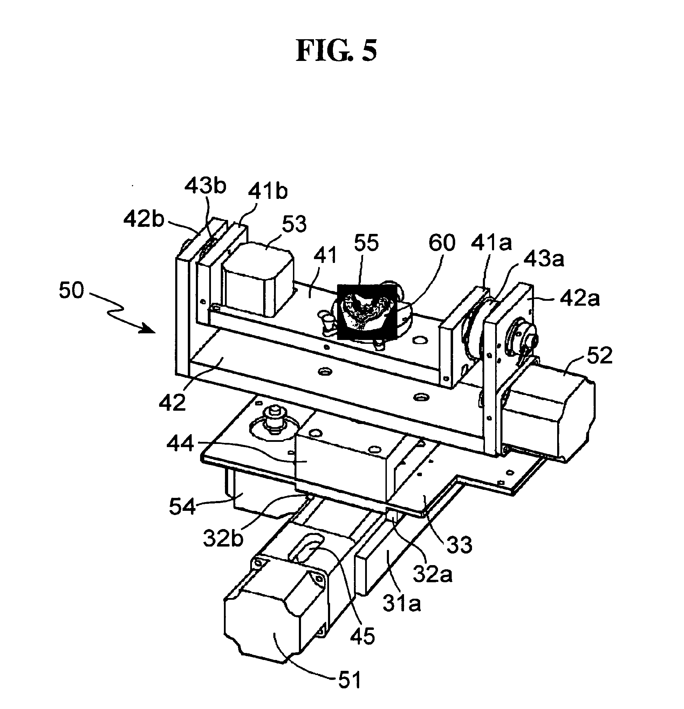

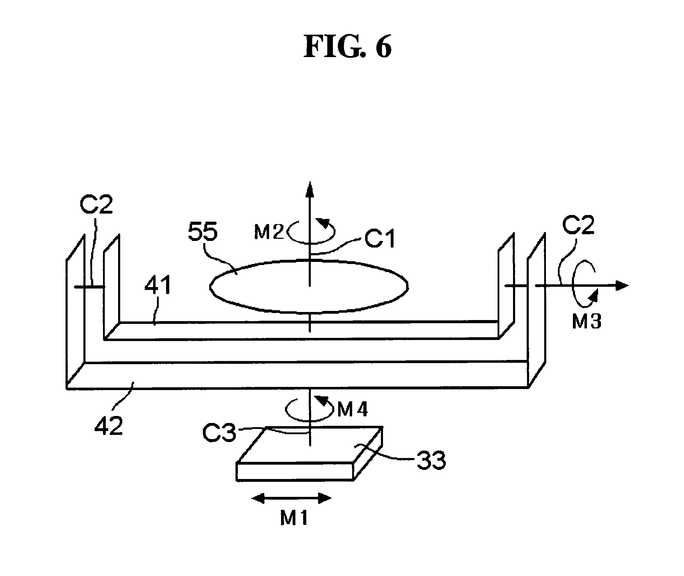

FIG. 4 is a schematic view showing the whole configuration of a four-axis three-dimensional scanning system for computer-aided modelling a tooth or teeth according to the present invention. FIG. 5 is an enlarged perspective view of a four-axis drive in the present invention system shown in FIG. 4. FIG. 6 is a view for explaining positional movement and posture alteration of a table jig in the four-axis drive of FIG. 5.

Referring to FIGs. 4 and 5, the four-axis 3D scanning system according to the present invention includes a controller 10, an image detector 20 and a four-axis drive 50, in order to measure configuration data of a tooth plaster model 60 necessary for tooth correction.

The tooth plaster model 60 alters its position and posture by the four-axis drive 50 at the state fixed on the table jig 55, and performs a scanning work for the tooth plaster model 60.

The four-axis drive 50 includes a rotation motor (M2) 53 which rotates the table jig 55 to make the tooth plaster model 60 rotate, a tilting motor (M3) 52 which tilts a tilting bed 41 supporting the table jig 55 and the rotation motor (M2) 53, a main rotation motor (M4) 54 which rotates a main rotation bed 42 supporting the tilting bed 41 and the tilting motor (M3) 52, and a linear motor (M1) 51 which linearly moves a linear drive bed 33 and a bed supporting frame 44 supporting the main rotation bed 42 and the main rotation motor (M4) 54.

The configuration of the four-axis drive 50 will be described in more detail with reference to FIGs. 5 and 6.

The table jig 55 is rotatably combined with the tilting bed 41 accommodating bearings in which the center axis C1 penetrates the lower surface of the tilting bed 41, and rotates around the center of the center axis C1 by a rotation motor (M2) 53. By this structure, the table jig 55 can smoothly rotate in the upper portion of the tilting bed 41.

Since the tilting bed 41 should perform tilting independently of the main rotation bed 42 which is located at the lower portion thereof, a pair of tilting supports 41a and 41b and a pair of main rotation beds 42a and 42b are vertically erected on both ends of the tilting bed 41 and the main rotation bed 42, respectively. Bearings 43a and 43b are supported to main rotation supports 42a and 42b between the tilting supports 41a and 41b and the main rotation supports 42a and 42b.

Also, the tilting center axes C2 extended from both ends of the tilting bed 41 are rotatably supported by the bearings 43a and 43b, respectively. The two center axes C2 which are located in both sides penetrates the respective circular holes on the main rotation supports 42a and 42b, and are extended so as to be coincident axially with each other. Simultaneously, the two center axes C2 are horizontal with the linear drive axis of the linear motor (M1) 51, and vertical with the rotation center axis C1 of the table jig 55.

The tilting center axis C2 is pivotally driven by the tilting motor (M3) 52 installed in the main rotation support 42a. When the tilting motor (M3) 52 rotates, the tilting bed 41 supporting the table jig 55 is tilted around the tilting center axis C2.

The main rotation bed 42 supporting the table jig 55 and the tilting bed 41 is rotatably supported to a bed support frame 44 through bearings in which the bed support frame 44 is formed in the form of a bridge on a linear drive bed 33 whose rotation center axis C3 is located in the lower portion thereof. The rotation center axis C3 is rotatably driven by the main rotation motor (M4) 54 installed on the linear drive bed 33.

When the tilting bed 41 and the main rotation bed 42 are parallel with each other, the rotation center axis C3 of the main rotation bed 42 is installed on the same line as the rotation center axis C1 of the table jig 55, and rotates independently of the rotation of the table jig 55.

The linear drive bed 33 is fixed to a pair of screw housings 32a and 32b which mounted on the lower portion of the linear drive bed 33. The screw housings 32a and 32b are mounted with a pair of linear guides 31a and 31b fixed to a base 30, respectively. The screw housings 32a and 32b are linearly driven by a screw 45, and linearly moves the linear drive bed 33.

The screw 45 is rotated by a linear motor 51. It is preferable that the base 30 is made of a high-weight material so that an optical system made of a laser diode 27 and a light receiving camera 29 is not influenced by vibration applied during driving of a motor in the drive 50 and external vibration, but maintains a stable posture. Also, in order to suppress vibration at maximum, it is preferable that a buffer playing a role of preventing vibration and made of rubber or silicon is mounted on the lower portion of the base 30.

Meanwhile, the linear motor 51, the tilting motor 52, the rotation motor 53, and the main rotation motor 54 are made of a step motor appropriately for successively transforming the position and posture of a tooth plaster model 60, respectively. A limit switch (not shown) is installed in each motor in order to limit a rotational angle within a predetermined distance range, and is driven by a motor drive 15 which is controlled by a controller 10.

As shown in FIG. 4, the controller 10 applies a control signal to the motor drive 15 and controls an amount of rotation of each motor 51-54 through the motor drive 15. Also, the controller 10 plays a role of extracting configuration data of a tooth plaster model 60 from the image signal input via a video board 25.

The controller 10 can be implemented using a well-known personal computer (PC) including a parallel port for interface with the motor drive 15.

The personal computer (PC) stores a model control program for controlling the position and posture of a tooth plaster model 60 by controlling an amount of rotation of each motor 51-54 via the motor drive 15, and a signal processing program for extracting configuration data of the tooth plaster model 60 from an image signal input via the video board 25, for example, in a storage device such as a hard disc.

An image detector 20 includes a laser diode 27 which irradiates laser slit light 21 onto a tooth plaster model 60, a camera 29, for example, such as a charge-coupled device (CCD) which photographs light which is irradiated by the laser diode 27 and reflected from the tooth plaster model 60, and a video board or image grabber 25 which grabs an image signal photographed by the camera 29.

Also, an upper support frame 35 coupling a pair of vertical frames 34a and 34b and the upper end of the pair of vertical frames 34a and 34b is installed on the base 30.

The laser diode 27 is installed perpendicularly with the upper support frame 35 located in the upper side of the tooth plaster model 60 so that laser slit light 21 is scanned in the vertical direction with respect to the tooth plaster model 60. The camera 29 connected to the video board 25 via a cable 28 is installed on the support frames 34b and 35 with a slope and a predetermined distance from the laser diode 27, so that laser slit light 22 reflected from the tooth plaster model 60 can be received.

In the case of the FIG. 4 embodiment, the light receiving direction of the camera 29 is set in the same direction as the movement direction of the linear motor 51. However, the main rotation bed 42 which supports the table jig 55 and the tilting bed 41 is rotationally driven around the rotation axis C3 by the main rotation motor (M4) 54. Accordingly, the installation angle of the camera 29 is not limited thereto.

Meanwhile, it is necessary to alter the position and measurement posture of the tooth plaster model sequentially so that each portion from one side to the other side of the tooth plaster model 60 can be scanned sequentially.

Since it is very difficult to determine a measurement posture when measuring a tooth plaster model, the standard tooth plaster model to be measured is modelled by the computer graphics using a three-dimensional scanner, and then a measurement process is simulated. Accordingly, a measurement angle and a measurement sequence which are accomplished through the linear motor 51, the tilting motor 52, the rotation motor 53 and the main rotation motor 54 are determined.

In this case, the standard tooth plaster model is read through a scanner and modelled using a computer. Then, while seeing the model on a monitor, a visible portion and an invisible portion are determined according to change in the relative position of the eyes. Accordingly, an angle through which the largest area is visible can be seen according to the shape of the tooth plaster model. Also, using the wide-area angle, a trial-and-error for finding the most appropriate angle when measuring the next tooth plaster model can be reduced.

In order to embody a three-dimensional scanning system, an automatic teaching system producing an optimal measurement posture path which is a path or a rotational angle through which the greatest amount of data can be obtained at a single scan, has been employed in which an open GL which is a computer graphics library which is a library for a three-dimensional graphics and modelling, and an expert system which adopts a method accessing a desired target through a deduction process based on a number of facts and variables, are combined with each other as is well-known, and thus an accuracy is enhanced and a non-measured portion is removed (see Korean Patent No. 382,905).

In order to use a four-axis driving system, the present invention has modified the existing measurement posture automatic teaching system as follows.

### 1. 3D scanner open GL simulation

Using an open GL which is a computer graphics tool, a completed four-axis driving three-dimensional shape measuring apparatus has been simulated on a virtual space.

### 2. Mesh generation of standard tooth plaster model

Three-dimensional point data of standard tooth plaster models of man and woman, the upper and lower jaws, existence and non-existence of a plaster base, and a coupling model is mesh generated, and then the mesh generated three-dimensional point data is put on the table jig of the virtual three-dimensional scanner.

### 3. Establishment of a target tooth to be measured

Here, since it is not possible to measure a complicated tooth model all at a time, a target tooth to be measured has been established as a portion of a tooth to be a target when measured in which a tooth model is divided into some portions, for example, a tongue-side, a lip-side, a coupling-side, and a narrow-side in a molar tooth.

### 4. Construction of a factbase

A factbase has been utilized as a reference of a system deduction according to the above-considered upper and lower jaws, existence and non-existence of a plaster base, and size of a bow of the jaw, based on a sex, an age, and existence and non-existence of a missing tooth or teeth of a person.

A measurement posture combining measured angle and position is determined through a deduction process using the factbase, and a measurement path which can measure a tooth plaster model within the shortest time is determined by combining a measurement posture according to each target tooth to be measured.

In the present invention, a table jig 55 on which a tooth plaster model 60 forms three kinds of the degree of freedom by rotation driving, tilting driving, and main rotation driving. Accordingly, the tooth plaster model 60 can be positioned in any posture. As a result, the modified automatic teaching system in the present invention has been able to adjust the automatic measurement ratio which conventionally remains 50% or so up more than 95%.

Hereinbelow, a process of measuring a three-dimensional coordinate according to the present invention will be described.

First, when a tooth plaster model 60 is fixed on a table jig 55, a linear motor 51, a rotation motor 53, a tilting motor 52, and a main rotation motor 54 are driven, to thereby return the table jig 55 to the origin 100. Then, the table jig 55 is moved to an initial position, and a Y-axis direction coordinate (a position of the linear drive bed 33) is stored.

Next, a predetermined target tooth starts to be measured along a predetermined optimal measurement posture path by the measurement posture automatic teaching system.

The motors M1-M4 are driven to change direction of the tooth plaster model 60, and measure a coordinate of a point to which laser slit light 21 is irradiated. Here, a current motor angle is measured to obtain a conversion matrix and the measure coordinate is automatically converted into a coordinate with respect to a reference coordinate system of teeth, to thereby measure each shape of teeth.

Hereinbelow, the position and posture conversion of the drive 50 by the motors M1-M4 will be described with reference to FIGs. 6 to 7B.

First, when a controller 10 applies a control signal including a direction control signal and a successive rectangular pulse to a motor drive 15 including four motor drives (not shown) via a parallel port, the motor drive 15 generates four divided rectangular pulses for controlling the motors M1-M4 each which includes a step motor and supplies the generated four rectangular pulses to the step motors, respectively.

As a result, the whole drive 50 linearly moves along a pair of linear guides 31 a and 31 b by the linear motor (M1) 51 to thereby determine the position of the tooth plaster model 60. Then, the table jig 55 is made to rotate around the rotation center axis C1 by the rotation motor (M2) 53.

Then, the tilting bed 41 on which the tooth plaster model 60 is mounted is tilted around the tilting center axis C2 by the tilting motor (M3) 52, and the whole drive 50 except for the linear motor (M1) 51 is made to rotate by the main rotation motor (M4) 54.

In this case, the present invention controls the four-axis motors M1-M4 to be driven so that a predetermined target tooth is measured along a predetermined optimal measurement posture path.

First, when the central portion of the tooth plaster model 60 is measured, the tilting bed 41 is tilted by the tilting motor (M3) 52 at the state where the tilting center axis C2 of the tilting bed 41 is maintained at the perpendicular state to the camera direction, as shown in FIG. 7A. As a result, the leading portion of the tooth of the tooth plaster model 60 supported to the table jig 55 contacts the laser slit light.

When the linear motor (M1) 51 moves forward or backward in this state, the tooth is measured from the upper portion to the lower portion with respect to the tooth width, or from the lower portion to the upper portion thereto.

Thereafter, when subsequent measurement is performed along the optimal measurement posture path, it is necessary to turn the tooth plaster model 60, so that the laser slit light 21 is scanned nearly perpendicularly to the measurement surface 2a of the tooth, and the laser slit light scanned on the measurement surface 2c is captured by the camera 29, in order to remove the problems of FIGs. 1A and 1C.

Accordingly, the rotation motor (M2) 53 rotates to thereby make the table jig 55 rotate by a predetermined angle. Also, the main rotation bed 42 supporting the table jig 55 and the tilting bed 41 is made to rotate by the main rotation motor (M4) 54 according to necessity, to thereby make the tilting bed 41 move by a predetermined angle in the camera direction.

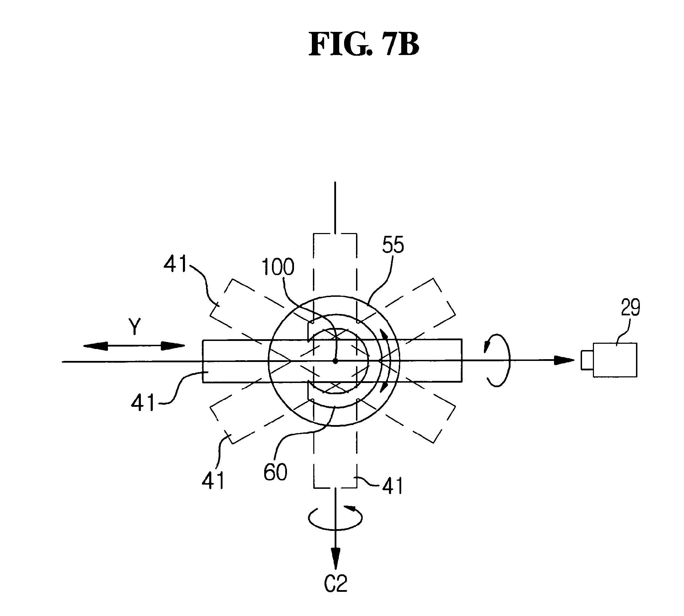

By repeating the above-described operation, when an inwardly slanted portion such as a tongue-side portion in a molar tooth which is located in both ends of the tooth plaster model 60 is measured, the main rotation bed 42 (that is, the tilting bed 41) supporting the table jig 55 and the tilting bed 41 is established in the same direction as or in the direction close to a direction which concatenates the camera 29 and the laser diode 27, by the main rotation motor (M4) 54, so that the laser slit light 21 can reach and be captured by the camera 29 as shown in FIG. 7B. When the tilting bed 41 on which the tooth plaster model 60 is mounted is tilted around the tilting center axis C2 (that is, the X-axis), a measurable posture for the molar tooth tongue-side portion is produced as shown in FIGs. 3A and 3B.

Thus, it is possible in the present invention to make the tilting center axis C2 of the tilting bed 41 rotate toward the camera capturing direction sequentially according to the measurement portions of the tooth plaster model 60. Accordingly, it is possible to measure the molar tooth tongue-side portion which is the most difficult to measure in the tooth plaster model 60 by an automatic measurement algorithm. As a result, almost all the portions of the tooth plaster model 60 can be automatically measured.

That is, since the three-dimensional scanner according to the present invention has a four-axis drive having four kinds of the degree of freedom, the tooth plaster model can be set to be positioned diagonally around the two axes. Thus, it is possible to scan the laser slit light and measure the scanned light by a camera. Therefore, the present invention has solved the conventional problems and simultaneously shorten a scan path remarkably.

The above-described embodiments have been described with reference to a computer-aided modelling with respect to a tooth plaster model as described above, but can be applied to other general object modelling at low cost, other than the tooth plaster model.

As described above, when a tooth plaster model for dental correction is scanned three-dimensionally, the present invention actively transforms and measures the position and posture of the tooth plaster model using a four-axis drive, to thereby enable the tooth plaster model to be transformed in any positions and postures, and thus provides an effect of measuring most of the positions which needs to be measured, including a molar tooth tongue-side portion accurately and quickly along the shortest scanning path, without any manipulation of an operator.

Also, the three-dimensional scanning system according to the present invention can automatically alter a tooth in any desired position and posture, through a four-axis drive. As a result, once an object to be measure is mounted and scanned, a user can measure the object without requiring to alter the posture of the object during scanning, to check the object individually during scanning, or to do any additional work. Accordingly, a measurement time can be shortened considerably, to thereby achieve an efficient scanning process. Since all portions of the object to be measured can be scanned, the object can be measured with a one-time process without requiring several numbers of scanning processes, to then guarantee accuracy.

As described above, a computer-aided tooth modelling three-dimensional scanning system according to the present invention has been described with reference to the accompanying drawings. However, the present invention is not limited to the above-described embodiments. It is apparent to one who has an ordinary skill in the art that there may be many modifications and variations within the same technical spirit of the invention.

## Claims

1. A driving apparatus for a three-dimensional (3D) scanning system, comprising:
a first rotation drive which rotatably drives a table jig on which an object to be measured is mounted;
a tilting drive which tilts the first rotation drive;
a second rotation drive which rotates the tilting drive independently of the first rotation drive; and
a linear drive which linearly moves the second rotation drive.

2. The driving apparatus of claim 1, wherein said first rotation drive comprises: a tilting bed which rotatably supports the table jig; and a first motor which is installed in the tilting bed for rotatably driving the object to be measured together with the table jig,
wherein said tilting drive comprises: a main rotation bed which supports the tilting bed so as to be tilted; and a second motor which is installed in the main rotation bed and tilts the tilting bed,
wherein said second rotation drive comprises; a support which rotatably supports the main rotation motor; and a third motor for rotating the main rotation bed, and
wherein the first through third motors are independently driven, respectively.

3. The driving apparatus of claim 1, wherein said object to be measured is a tooth plaster model.

4. A driving apparatus for a three-dimensional (3D) scanning system, comprising:
a tilting bed which rotatably supports a table jig on which an object to be measured is mounted, around a first rotation center axis and includes first and second tilting supports which are vertically installed on both ends thereof;
a rotation motor which is installed in the tilting bed for rotatably driving the table jig;
a main rotation bed which includes first and second main rotation supports which are vertically installed in correspondence to the first and second tilting supports on both ends thereof and supports first and second tilting center axes extended from the first and second tilting supports so as to be tilted;
a tilting motor which is installed in the main rotation bed and tilts the tilting bed;
a support frame which rotatably supports the main rotation bed around a second rotation center axis;
a linear drive bed on which the support frame is installed;
a main rotation motor which is installed on the linear drive bed and rotatably drives the main rotation bed; and
a linear drive which linearly moves the linear drive bed,
wherein the table jig, the tilting bed and the main rotation bed are independently driven, respectively.

5. The driving apparatus of claim 4, wherein said first and second tilting center axes coincide with each other, and are horizontal with respect to a linear drive axis of the linear drive, and wherein said linear drive axis is perpendicular with the first rotation center axis of the table jig.

6. The driving apparatus of claim 4 or 5, wherein said second rotation center axis of the main rotation bed is set to be positioned on the same line as the first rotation center axis of the table jig when the tilting bed and the main rotation bed are parallel with each other.

7. A three-dimensional scanning system for a computer-aided tooth modelling, comprising:
a table jig on which a tooth plaster model is mounted;
a laser light source which irradiates laser light to the tooth plaster model;
a camera which photographs light reflected by the laser irradiated from the laser light source;
a drive having a four axes to alter position and posture of the tooth plaster model; and
a controller which controls the four-axis drive to alter the position and posture of the tooth plaster model, and signal-processes the image signal photographed by the camera to produce configuration data,
wherein the four-axis drive comprises: a first rotation drive which rotatably drives a table jig on which an object to be measured is mounted; a tilting drive which tilts the first rotation drive; a second rotation drive which rotates the tilting drive independently of the first rotation drive; and a linear drive which linearly moves the second rotation drive.

8. A three-dimensional (3D) scanning system for a computer-aided tooth modelling, comprising:
a table jig on which a tooth plaster model is mounted;
a tilting bed which rotatably supports the table jig around a first rotation center axis, and includes first and second tilting supports which are vertically installed on both ends thereof;
a rotation motor which is installed in the tilting bed for rotatably driving the table jig;
a main rotation bed which includes first and second main rotation supports which are vertically installed in correspondence to the first and second tilting supports on both ends thereof and supports first and second tilting center axes extended from the first and second tilting supports so as to be tilted;
a tilting motor which is installed in the main rotation bed and tilts the tilting bed;
a support frame which rotatably supports the main rotation bed around a second rotation center axis;
a linear drive bed on which the support frame is installed;
a main rotation motor which is installed on the linear drive bed and rotatably drives the main rotation bed;
a linear drive which linearly moves the linear drive bed;
a laser light source which irradiates laser light vertically when the tooth plaster model is located at the origin;
a camera which photographs laser light reflected from the tooth plaster model; and
a controller which controls the motors to alter the position and posture of the tooth plaster model, and signal-processes the image signal photographed by the camera to produce configuration data,
wherein the table jig, the tilting bed, the main rotation bed and the linear drive bed are independently driven, respectively.

9. The 3D scanning system of claim 8, wherein said first and second tilting center axes coincide with each other, and are horizontal with respect to a linear drive axis of the linear drive, and wherein said linear drive axis is perpendicular with the first rotation center axis of the table jig.

10. The 3D scanning system of claim 8, wherein said second rotation center axis of the main rotation bed is set to be positioned on the same line as the first rotation center axis of the table jig when the tilting bed and the main rotation bed are parallel with each other.

11. The 3D scanning system of claim 8, wherein when the camera is set to be positioned in the upper portion in the same direction as the linear drive axis of the linear drive bed with a predetermined distance from the laser light source, the molar tooth tongue-side surface of the tooth plaster model can be measured at the state where the tilting center axis of the tilting bed is set by the main rotation motor to have a posture in the same direction as or close to a camera light receiving direction and the tilting bed is tilted.
